# EUROPEAN PATENT SPECIFICATION

(11) **EP 1 793 868 B1**
(45) Date of publication and mention of the grant of the patent: **29.12.2010**
(21) Application number: 05801399.6
(22) Date of filing: 23.09.2005
(51) Int. Cl.: A61K 49/18

(54) **LIPOSOMAL CONTRAST AGENTS FOR CEST IMAGING**
LIPOSOMALE KONTRASTMITTEL FÜR DIE CEST-BILDGEBUNG
AGENTS DE CONTRASTE LIPOSOMAUX POUR TECHNOLOGIE D'IMAGERIE CEST

(30) Priority: 23.09.2004 US 612173 P
(43) Date of publication of application: 13.06.2007
(73) Proprietor: GUERBET, 93420 Villepinte (FR)
(72) Inventor: PORT, Marc, F-95170 Deuil la Barre (FR)
(74) Representative: Warcoin, Jacques
(86) International application number: PCT/EP2005/054798
(87) International publication number: WO 2006/032705

(56) References cited:
- EP-A- 1 331 012
- EP-A- 1 466 629
- WO-A-02/43775
- WO-A-90/04943
- WO-A-96/25955
- WO-A-2004/023981
- WO-A-2006/114738
- WO-A2-2006/114738
- US-A- 5 534 241
- US-A- 6 045 821
- US-B1- 6 569 451
- HEVERHAGEN J T ET AL: "Encapsulation of gadobutrol in AVE-based liposomal carriers for MR detectability" MAGNETIC RESONANCE IMAGING 2004 UNITED STATES, vol. 22, no. 4, May 2004 (2004-05), pages 483-487, XP002398083 ISSN: 0730-725X
- FOSSHEIM S L ET AL: "Paramagnetic liposomes as MRI contrast agents: influence of liposomal physicochemical properties on the in vitro relaxivity." MAGNETIC RESONANCE IMAGING. JAN 1999, vol. 17, no. 1, January 1999 (1999-01), pages 83-89, XP002398084 ISSN: 0730-725X
- CARAVAN P ET AL: "GADOLINIUM(III) CHELATES AS MRI CONTRAST AGENTS: STRUCTURE, DYNAMICS, AND APPLICATIONS" CHEMICAL REVIEWS, ACS,WASHINGTON, DC, US, vol. 99, no. 9, September 1999 (1999-09), pages 2293-2352, XP000852435 ISSN: 0009-2665
- AIME SILVIO ET AL: "Highly sensitive MRI chemical exchange saturation transfer agents using liposomes." ANGEWANDTE CHEMIE (INTERNATIONAL ED. IN ENGLISH) 26 AUG 2005, vol. 44, no. 34, 26 August 2005 (2005-08-26), pages 5513-5515, XP002398010 ISSN: 0570-0833
- MAIOCCHI A: "THE USE OF MOLECULAR DESCRIPTORS IN THE DESIGN OF GADOLINIUM (III) CHELATES AS MRI CONTRAST AGENTS" MINI REVIEWS IN MEDICINAL CHEMISTRY, BENTHAM SCIENCE PUBLISHERS, HILVERSUM, NL, vol. 3, no. 8, 1 December 2003 (2003-12-01), pages 845-859, XP008068785 ISSN: 1389-5575
- MAIOCCHI A.: 'THE USE OF MOLECULAR DESCRIPTORS IN THE DESIGN OF GADOLINIUM (III) CHELATES AS MRI CONTRAST AGENTS' MINI REVIEWS IN MEDICINAL CHEMISTRY, BENTHAM SCIENCE PUBLISHERS, HILVERSUM, NL vol. 3, no. 8, 01 December 2003, pages 845 - 859, XP008068785 ISSN: 1389-5575
- CARAVAN P. ET AL: 'GADOLINIUM(III) CHELATES AS MRI CONTRAST AGENTS: STRUCTURE, DYNAMICS, AND APPLICATIONS' CHEMICAL REVIEWS, ACS,WASHINGTON, DC, US vol. 99, no. 9, 01 September 1999, pages 2293 - 2352, XP000852435 ISSN: 0009-2665

## Description

The invention refers to contrast agents encapsulating systems, liposomes carrying lanthanides chelates, for CEST imaging.

These systems are useful for the in vivo or in vitro determination of physico-chemical parameters of diagnostic interest.

It is well known that the potential of Magnetic Resonance Imaging (MRI) procedures can be further enhanced when this diagnostic modality is applied in conjunction with the administration of contrast agents. Many contrast agents, i.e. chemicals able to promote marked changes in the relaxation rates of the tissue protons, have been described, particularly T1 agents represented by paramagnetic complexes, mostly containing GD(III) or Mn(II) ions. These complexes affect the relaxation rates of the bulk water through the exchange of the water molecules in their coordination spheres (Caravan P, et al. Chem. Rev 1999, 99:2293-2352; the Chemistry of Contrast Agents in Medical Magnetic Resonance Imaging. Chichester, UK: John Wiley & Sons; 2001. p 45-120). The proton relaxivity is a measure for the ability of the paramagnetic substance to accelerate the nuclear relaxation of water protons in the media where this paramagnetic substance has been dissolved.

A different approach in order to efficiently reduce the water signal has been shown to occur when a proper radiofrequency (rf) irradiating field is applied at the resonance frequency of the exchangeable protons saturating it. This results in a net decrease of the bulk water signal intensity owing to a saturation transfer effect. Balaban and co workers named this contrast-enhancing procedure Chemical Exchange Dependent Saturation Transfer (CEDST or, more commonly, CEST) (Balaban RS.: Young IR, editor. Methods in Biomedical Magnetic Resonance Imaging and Spectroscopy. Chichester, UK: John Wiley & Sons; 2000. Vol.1. p 661-6667). A good contrast agent for CEST imaging has to possess mobile protons, whose exchange rate with water is as high as possible before their broadening makes the rf irradiation ineffectual. Larger chemical shift differences enable the exploitation of faster exchange, resulting in an enhanced CEST effect.

More precisely, a CEST contrast agent is such that the resonance of its mobile protons is such that ωΔ/ k1 >> 1 with
k1 = water exchange rate, Δω = frequency shift between the mobile protons of the CEST contrast agent and of the bulk water.

A Δω more than 2 ppm is preferable in order not to irradiate the bulk water. A high Δω allows the use of a large irradiating zone, and minimises the transfer saturation effects between endogenous water and bulk water.

Two categories of CEST agents have been described in the prior art.

### a) diamagnetic agents.

WO 00/66180 describes compounds that are diamagnetic organic molecules such as sugars, amino acids, nitrogen-containing heterocycles, purines, guanidine, nucleosides, imidazole and derivatives thereof, barbituric acid and analogous thereof, wherein heterocyclic compounds having exchangeable OH or NH groups such as 5,6- dihydrouracil, 5-hydroxytryptophan are particularly preferred when the pH is determined according to claimed method.

These agents allow to increase the number of the protons of the contrast agent that can be exchanged.

The diamagnetic systems are advantageously endowed with short relaxation rates. Unfortunately, however, the chemical shifts of exchangeable protons thereof are only slightly shifted (1-5 ppm) from bulk water signal and, therefore, slower exchange rate can be exploited before coalescence takes place.

### b) paramagnetic agents

Sherry et al. showed that a particularly useful source of highly shifted exchangeable protons can be provided by the slowly exchanging water protons bound to a paramagnetic Eu(III)-chelate. In this complex, the irradiation of such protons, which resonate at 50 ppm downfield from the bulk water signal, determined a significant CEST effect in the images obtained at 4.7 T. (Sherry et al. in J Am Chem. Soc 2001, 123:1517-1518). However no contrast is detectable if the uptake between the target and the surrounding tissue is similar. Moreover, these contrast agents, in general, allow the production of images of the targeted tissue or organ but they all are unable to measure the metabolic conditions of the examined tissue and to refer about the physico-chemical parameters determining thereof.

Improvements of paramagnetics agents for CEST imaging are described in EP 1 331 012 presenting chelates which stucture has been modified such as macrocyclic tetra-amide derivatives of the 1,4,7,10-tetraazacyclododecane-1,4,7,10-tetraacetic acid (DOTA) or of the tris-amide derivatives of the 1,4,7,10-tetraazacyclododecane-1,4,7-triacetic acid (DO3A), and notably the chelates of the 1,4,7,10-tetraazaciclododecane-1,4,7,10-acetic acid tetraglycineamide (DOTAM-Gly). This document describes for increasing the CEST effect notably the use of a paramagnetic contrast agent comprising a single CEST molecule endowed with both the two magnetically non equivalent labile protons pools.

In such compounds of EP 1 331 012, the mobile protons studied are the water protons linked to the lanthanide in the inner sphere of the chelate and/or the protons of amides linked in the first coordination sphere of the lanthanide. The value Δω is better with these agents.

In particular, the DOTA tetraamides are designed in order to have a low water exchange (water linked in the inner sphere) and to use the amides protons (linked in the inner sphere). It is reminded that the inner sphere corresponds to the water molecules that are in contact with the metal ion in the complex and that are in fast exchange with the bulk water molecules

But such products may still lack of sensibility or imply difficulties in terms of imaging techniques (notably irradiation to apply to each scan).

The patent application WO 02/43775 discloses paramagnetic chelates of DOTAM or of DOTAM derivatives like DOTAM-Gly with a lanthanide for CEST imaging and for measuring by MRI a chemico-physical parameter of diagnostic interest. However, this document does not describe liposome or double emulsion as encapsulating systems for mobile shifted protons.

EP 1 466 629 which is a document according to Article 54(3) EPC describes substrates adducts [SR-SH] for Cest imaging, that may be compartimentalized in a liposome in which SR is Ln [DOTP]⁴⁻ wherein Ln is a lanthanide selected from Cerium (III), Praseodymium (III), Neodymium (III), Dysprosium (III), Erbium (III), Terbium (III), Holmium (III), Thulium (III), Ytterbium (III) and Europium (III) and SH is a diamagnetic substrate selected from linear and cyclic polyamines, polyaminoacids, proteins, polysaccharides, polyamidoamines, peramidated polyaminoacids, dendrimers containing amide groups, polycyclodextrins, polysaccharides and alginates.

The applicant has now identified a totally different approach for obtaining a very satisfying sensibility of the CEST imaging. Indeed the applicant has studied products exhibiting a pool with a very high number of mobile protons. This pool is obtained with an encapsulating system (ES), being liposomes , that encapsulate the mobile protons.

The water molecules encapsulated in the encapsulating system:
- are at the contact with a shift agent such as a lanthanide complex (typically a complex of a chelate with a lanthanide) encapsulated in the encapsulating system or inserted in the lipophilic layer of the encapsulating system
- and represent the mobile protons to irradiate.

More precisely, in the prior art, there is a water exchange between the bulk and the lanthanide complex (complex of a chelate and a paramagnetic metal such as Eu) of one H20 molecule for one chelate molecule.

With the invention, for one paramagnetic metal ion, there is a water exchange of a very high number of H20 molecules (one million for instance) between the interior of the encapsulating system and the bulk. Due to the structure and the concentration of the shift agent, the resonance of the water protons inside the encapsulating system is shifted from that of the external water. Then, the NMR spectra ¹H of the ES solution obtained show one signal for the water of the solution outside the encapsulating system and one separated signal for the water inside the encapsulating system. A very satisfying CEST effect is obtained by a good choice of the paramagnetic metal that determines Δω and the structure of the encapsulating system that determines the k₁. This allows to have for one paramagnetic metal ion (and one chelate molecule) administered to the patient a much higher signal compared to the prior art. Best results can be obtained with different appropriate combinations of metal and chelate like those described hereafter. Indeed, prior systems like polymeric or dendrimeric molecules having a high number of water molecules (exchangable protons) need high energy (a high magnetic field B1) to reach the saturation. In the prior art, a high energy, function of the Δω, was required to obtain the saturation for the protons which display a "dispersed" behaviour on the NMR spectra (large area): it was particularly true for amine protons of the large molecules, due notably to their position in the molecule and to their physico chemical interactions. This high energy was until now the main disadvantage of CEST imaging.

On the opposite, with the encapsulating systems of the invention, the energy required is much lower as described hereafter. This would be due to the low dispersion of the shift and to the water exchange rate of the encapsulating systems: the protons behave similarly in the encapsulating systems.

Thus according to a first aspect the invention relates to the use of a contrast agent in a method for CEST imaging wherein said contrast agent comprises a proton pool Encapsulating System (ES) that contains a pool of water mobile shifted protons as claimed in claim 1 and in claim 2.

Said contrast agent comprises a proton pool encapsulating system that contains 1) a pool of water mobile protons to be shifted and 2) a shift agent.

Said encapsulating system ES is any encapsulating system with water entrapped, being a liposome.

It is not necessary to use a cest agent (and thus the metal) entirely included within the encapsulating system. The agent can have only a part within the encapsulating system, and an other part not entrapped part (at least a part located inside the lipid layer or even outside) since this latter part implies only a low shift of the bulk water. It is for instance possible to covalently link the cest agent (the chelate) to a phospholipid inserted in the encapsulating system. For this, the chelate is coupled, eventually with a linker, to a lipophilic chain of a phospholipid of the ES membrane. The protons of the protons pool are shifted due to the shift agent. The shift agent is a paramagnetic complex of a lanthanide.

Said lanthanide is chosen among Erbium (II), Europium (III), Dysprosium (III), Gadolinium (III), Praseodymium (III), Neodymium (III), Terbium (III), Holmium (III), Thulium (III), Ytterbium (III)

According to a realisation the encapsulating system is of high water exchange, notably k₁ = water exchange rate = 10 to 10 E 6 s-1

According to a realisation the ES (the liposomes) forming lipids comprise phospholipids or hydrogenated phospholipids or derivatives thereof among phosphatidylcholines (lecithins) (PC), phosphatidylethanolamines (PE), lysolecithins, lysophosphatidylethanolamines, phosphatidylserines (PS), phosphatidylglycerols (PG), phosphatidylinositol (PI), sphingomyelins, cardiolipin, phosphatidic acids (PA), fatty acids, gangliosides, glucolipids, glycolipids, mono-, di or triglycerides, ceramides or cerebrosides.

Advantageously, a mixture of saturated and unsaturated phospholipids and of cholesterol is used, notably in the proportion 40/10/50 to 60/5/35 for instance 55/5/40.

Advantageously the ES is a liposome formed with phospholipids having an intermediate HLB value in the order of 10.

According to a realisation the ES (the liposomes) are of diameter of range 20-5000 nm.

According to a realisation the ES (the liposomes) structure is adapted for blood pool imaging.

According to a realisation the ES (the liposomes) structure is adapted for specific targeting. The specific targeting refers to the targeting of a biological target such as a tissue or a pathologic area, recognized specifically by the contrast agent. The encapsulating system comprises a least a targeting biovector and are thus very useful for molecular imaging.

The invention also relates to the use of a pharmaceutical composition comprising a physiological acceptable carrier and a contrast agent as described above.

According to another aspect, the invention relates to:
- A method of CEST imaging wherein a CEST contrast agent as described above.
- A method for the determination by MRI of a chemico-physical parameter in a human or animal body organ, fluid or tissue, wherein a CEST contrast agent is employed whose saturation transfer capability is correlated to the chemico-physical parameter of interest and a CEST MRI image for this chemico-physical parameter is registered, wherein said contrast agent is an agent as described above
- A diagnostic method of a chemico-physical parameter comprising the administration in appropriate quantity of at least one contrast product comprising a pool of mobile protons in chemical exchange with the water medium protons and able, when a proper radiofrequency rf irradiating field is applied at the resonance frequency of the said exchangeable protons pool, to generate a saturation transfer effect between at least a part of said mobile pool of protons and the water protons and wherein said saturation transfer relates to the chemico-physical parameter of interest.

Liposomes encapsulating lanthanides chelates are known but the prior art neither describes nor suggests to use of liposome technology for CEST imaging. Further the liposomes have to be appropriate for the CEST imaging.

### Detailed description

An encapsulating system ES refers to liposomes for convenience the term ES/liposomes is also used.

The liposomes are spherical vesicles having a lipid layer surrounding a central space. The present invention is particularly concerned with unilamellar and multilamellar liposomes which respectively have a single lipid bilayer or multiple lipid bilayers surrounding an aqueous core. Liposomes comprising multiple lipid bilayers are also called spherulites.

Liposomes spontaneously form upon dispersion of lipids, particularly phospholipids, in aqueous media and the liposomal structure of the agents of the invention can be produced by conventional techniques. Such conventional techniques are referred to in WO92/21017 (Unger) and by Papahadjopolous in Ann Rep. Med. Chem. 14: 250-260 (1979) and include reverse evaporation, freeze-thaw, detergent dialysis, homogenization, sonication, microemulsification and spontaneous formation upon hydration of a dry lipid film. Multi-lamellar liposomes can be used according to the invention or may be converted to liposomes with lower lamellarity, or to unilamellar liposomes, by known methods. Unilamellar liposomes can also be prepared directly.

Liposome preparations are typically heterogeneous in size and the liposomes used according to the invention may be sized to the desired diameter by known techniques, eg. extrusion, freeze-thaw, mechanical fragmentation, homogenization and sonication. The liposomes used according to the invention are advantageously 20-5000 nm diameter, unilamellar or multi-lamellar.

The ES/liposomes may be lyophilized to increase shelf life and lyophilized ES/liposomes may be reconstituted by vigorous shaking with aqueous buffer prior to use. Formulations may include agents which serve to stabilize the ES/liposomal material for the lyophilization procedure.

Liposomes smaller than 200 nm may be sterilized after formulation by filtration. The lipids used as the liposomal membrane forming molecules and more generally as the ES membrane forming molecules are typically phospholipids or hydrogenated phospholipids such as natural or synthetic phosphatidylcholines (lecithins) (PC), phosphatidylethanolamines (PE), lysolecithins, lysophosphatidylethanolamines, phosphatidylserines (PS), phosphatidylglycerols (PG), phosphatidylinositol (PI), sphingomyelins, cardiolipin, phosphatidic acids (PA), fatty acids, gangliosides, glucolipids, glycolipids, mono-, di or triglycerides, ceramides or cerebrosides, eg. liposome membrane forming compounds such as are described in WO-92/21017.

The membrane forming lipids may also comprise polymerizable lipids, e.g. methacrylate lipids, thiol and disulphide lipids, dienoate lipids, styryl lipids and diacetylanic lipids as described by Johnston in Liposome Technology Vol. I, Gregoriades Ed., pages 123-129 (1983) and Singh in Phospholipid Handbook, Cevc Ed., Dekker, pages 233-291 (1993) and references therein. The use of polymerizable lipids in the formation of the liposomes provides one route for increasing liposome stability

The ES and liposomal membrane can also have steroids and other compounds incorporated into it, eg. to affect the biodistribution of the liposome. Suitable steroids include for example cholesterol, cholesterol derivatives, cholestane, cholic acid, and bile acids, but particularly cholesterol.

The biodistribution modifiers can be incorporated by the use of a phospholipid derivative having a pendant biodistribution modifying function, by the use of a biodistribution modifying agent having a hydrophobic "anchor" moiety which associates with the ES or liposomal membrane or by coupling a biodistribution modifier to an anchor molecule (such as discussed above in relation to chelate tethering) present in the liposomal membrane.

Particularly preferred biodistribution modifiers, also called furtive agents, include compounds, especially amphiphilic polymers, which serve to reduce in vivo protein binding to the liposome and thus prolong the half life of the liposomes in the blood. Polyalkyleneoxy polymers, such as polyethylene glycol (PEG) and gangliosides, such as Gm.sub.1, are effective in this regard.

Incorporation of 1-10%, relative to the weight of liposome membrane forming material, of PEG-PE derivatives significantly thus extends blood half life. Liposomes prepared from perfluorinated phospholipids (see Santaella, FEBS Letters 336: 481-484 (1993) and Angew, Chem. Int. Ed. Eng. 30: 567-568 (1991)) can also extend blood half-lives.

Double emulsions which do not form part of the present invention refer to emulsions water/oil/water being dispersions of oily globules in which water drops have been prior dispersed.. Advantageously, two surfactants used for the double emulsion are such that their respective HLB allows the formation of the globules, one surfactant with high HLB, the other surfactant with low HLB. The ratio is such that the efflux of water can be controlled between the inside and the outside of the globules. Double emulsions are for instance described in "how does release occur?" Pays K, Giermanska-Kahn J, Pouligny B, Bibette J, Leal-Calderon F, J Control Release. 2002 Feb 19;79(1-3):193-205, and in "Double emulsions: a tool for probing thin-film metastability" Pays K, Giermanska-Kahn J, Pouligny B, Bibette J, Leal-Calderon F, Phys Rev Lett. 2001 Oct 22;87(17):178304.

In order to maximise the quantity of chelates within the ES, appropriate protocols may be used, such as those detailed hereafter.

It is also possible to use chemically modified chelates which are incorporated at least partially in the ES membrane. Such lipophilic chelates are described in US 5 804 164, US 5 460 799, WO91/14178. More precisely the coupling can be made through a group - (CH2)ₐ -OO NR₁R₂, with :
a. a is 1, 2 or 3
b. R₁, R₂ represent independently a chain C₇-C₃₀, substituted or not, linear or branched, eventually interrupted by O, NH, R₃ or S, where R₃ is a C₁-C₃ alkyl
c. Or R₁, R₂ represent independently a group 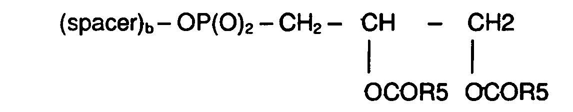 with b =0 to 2 and R₅ is a saturated or unsaturated group of at least 6 carbon atoms.

In order to incorporate the shift agent inside the liposome ie in the inner layer of the liposome, one can use liphophilic complexes such as Dy3+ or Tm3+ DTPA BC14A to be incorprated in the liposome. After preparation of the liposomes, the complexes inserted in the outer layer are transmetallated with a non shift metal agent such as La3+.The transmettallation by a T1 or T2 relaxer metal (for example Gd3+) can provide multimodal CEST/T1 MRI contrast agent.

Spacer may include a part issued from a phosphoglyceride, for exemple a CH₂CH₂ from a phosphodiglyceride such as phosphatidyl ethanolamine. Spacer may include groups derivated from peptides, pseudopeptides, polyalkylene glycols, PEG and analogues. In such ES systems, the chelate may be present.

ES/Liposome biodistribution is also significantly dependent upon surface charge and the liposomes according to the invention may desirably include 1 to 10%, relative to the weight of liposome membrane forming material, of negatively charged phospholipids such as for example phosphatidylserine, phosphatidylglycerols, phosphatidic acids, and phosphatidylinositol.

Liposomes for encapsulating lanthanides chelates complexes are described for instance in EP 314 764, WO 9625955 and WO2004023981.

The use of dispersions of microvesicles containing concentrated solutions of paramagnetic species encapsulated in the vesicles e.g. liposomes as carriers of NMR contrast agents is described in EP 314 764 which discloses injectable aqueous suspensions of liposomal vesicles carrying encapsulated at least one organic.

Several improvements have been described in order to avoid unwanted elimination of the liposomes in the body fluids, namely the liver and spleen such as below.
- 1) coating liposomes with copolymers containing hydrophilic and hydrophobic segments
- 2) incorporation of protective substances in the vesicle forming lipids (EP-A-0 354 855, WO-A-91/05545)
- 3) incorporating, as "stealth" factors, to the vesicle forming lipids of products such as palmitoylglucuronic acid (PGlcUA) in order to improve the half-life of liposomes in the blood
- 4) using agents for inhibiting adsorption of protein on the liposome surface comprising a hydrophobic moiety at one end and a hydrophilic macromolecular chain moiety on the other end. The preferred hydrophobic moieties are alcoholic radicals of long chain aliphatic alcohol, a sterol, a polyoxypropylene alkyl or a glycerine fatty acid ester and phospholipids while preferred hydrophilic moieties are polyethylen glycols (PEG). Non-ionic surface active agents in which PEG and an alcoholic radical of the hydrophobic moiety are bound by ether bond or
   PEG-bound phospholipids are particularly preferred. Upon formation the agent is admixed with liposome forming phospholipids to produce "stealth" liposomes.
- 5) Lowering the the size of the liposomes: the lifetime of liposomes in the blood may be significantly prolonged by making the vesicles very small, i.e. making them less size-recognisable by opsonin.
- 6) Adjusting the liposome suspensions composition such as described in WO9625955 in which:
   (a) the liposome forming lipids comprise between 80 and 99 mole % of neutral phospholipids and from about 1 to 20 mole % of negatively charged phospholipids, whose phosphatidyl moiety is linked to glycerol,
   (b) (b) at least 80% (by volume) of the liposome vesicles are in the 0.2-1.0µm range, and
   (c) depending on the liposome size the lipid concentration(CLip) in the suspensions is below 20 mg/ml for liposomes with average diameter of 1.0µm and below 100 mg/ml for liposomes with average diameter of 0.2 µm.

In these liposome suspensions described in WO9625955, the neutral phospholipids comprise the usual saturated and unsaturated phosphatidylcholines and ethanolamines, for instance, the corresponding mono- and di-oleoyl-, mono-and di-myristoyl-, mono- and di-palmitoyl-, and mono- and di-stearoyl- compounds. The negatively charged phospholipids comprise the phosphatidyl glycerols preferably dimyristoylphosphatidyl glycerol (DMPG), dipalmitoylphosphatidyl glycerol (DPPG), distearoylphosphatidyl glycerol (DSPG) and optionally the corresponding phospholipids where the glycerol is replaced by inositol. In addition, the lipids of the liposomes may contain additives commonly present in liposome formulations, like the sterols and some glycolipids; the sterols may include cholesterol, ergosterol, coprostanol, cholesterol esters such as the hemisuccinate (CHS), tocopherol esters. The glycolipids may include cerebrosides, galacto-cerebrosides, glucocerebrosides, sphingo-myelins, sulfatides and sphingo-lipids derivatized with mono-, di- and trihexosides.

According to the parameters to study by MRI, ES/liposomes may be such as those described in WO2004023981 which describes liposomes not sensitive to the biological environment and liposomes that are sensitive to the biological environment. An envirosensitive liposome can comprise, for example, a thermosensitive liposome, a pH-sensitive liposome, a chemosensitive liposome and a radiation-sensitive liposome.
- A non-sensitive liposome can comprise, for example, DSPC/Cholesterol (55: 45, mol :mol). A thermosensitive liposome can comprise, for example, a formulation selected from the group consisting of DPPC-PEG2000,DPPC-DSPE-PEG2000 (95: 5, mol : mol) ;DPPC-MSPC-DSPE-PEG2000 (90: 10: 4, mol :mol).
- "Envirosensitive liposome" means a liposome formulated using physiologically compatible constituents, such as dipalmitoylphosphatidyl-choline and dipalmitoylphosphatidyl-glycerol phospholipids.

For instance, thermosensitive liposomes can be formed from a combination of lipids that comprises :
- dipalmitoylphosphatidylcholine- polyethylene glycol (DPPC-PEG2000)
- dipalmitoylphosphatidylcholine- distearoylphosphatidylethanolamine-polyethylene glycol (DPPC-DSPE-PEG2000) (95: 5, mol : mol)
- polyenylphosphatidylcholine-MSPC- distearoylphosphatidylethanolamine-polyethylene glycol (DPPC-MSPC-DSPE-PEG2000) (90: 10: 4, mol :mol).

Liposomes may be as described in US 6 045 821 which presents liposomal agents in which metal chelate moieties are tethered to the liposomal membrane, in particular using macrocyclic chelant moieties having a lipophilic anchor group attached at only one ring atom, the macrocyclic chelant and anchor groups preferably being coupled to each other, advantageously via a biodegradable bond, after liposome formation.

These liposomes are presented as better than their prior art :
- Membrane tethered chelates involving linear chelant groups, such as DTPA, with one or two of the chelating functions derivatized to attach to lipophilic anchor groups: linear chelants carrying twin lipophilic anchor groups.

These liposomal agents will generally include, besides the anchor/chelate molecules, liposome membrane forming compounds, i.e. lipids and in particular phospholipids, as well as the materials which make up the liposome core and its external environment, generally in each case an aqueous medium. The chelated metals are tethered internally (and eventually also externally) to the liposomes in several ways, for example:
(i) by metallation of chelant groups tethered to the surface of preformed liposomes;
(ii) by coupling chelate moieties to anchor molecules in preformed liposomes;
(iii) by forming liposomes using a lipid mixture including chelate anchor molecules.

The ES liposomes may comprise phospholipids with short acyl chain lengths such as DMPC (dimyristoyl phosphatidyl choline), DPPC (Dipalmitoylphosphatidylcholine), DPPG (Dipalmitoylphosphatidylglycerol) and DMPG (dimyristoyl phosphatidyl glycerol).

Considering the optimisation of the water transfer through the ES/liposomes, the ES/liposomes composition may advantageously be adapted in order to adjust the water exchange property of the liposomes. Such adaptation is described notably in International Journal of Pharmaceuticals, 233, 2002,131-140, Glogard et al. In particular a high level of cholesterol may rigidify the liposomes membrane and decrease the water exchange across the membrane. A reduced surface area-to-volume ratio and the presence of multilamellar bilayers will slow down the water exchange between the liposome interior and exterior.

In the embodiment referring to CEST molecular imaging, active targeting to specific organs or tissues can be achieved by incorporation of lipids with attached thereto biovectors such as monoclonal antibodies or antibody fragments that are specific for tumour associated antigens, lectins or peptides. Targeting liposomes ES are notably described in US 6 350 466 wherein the liposomes comprise a targeting agent attached to hydrophilic head groups of a portion of lipids in the lipid sheet. Biovectors may be appropriately coupled with lipophilic groups allowing the insertion into the ES membrane such that the biovector is at least displayed on the external face of the ES.

Targeting delivery of imaging agents by liposomes is also well described in Handbook of Targeting delivery of imaging agents, Ed Wladimir P.torchilin, Massachusetts, 1995, pages 149-155 and 403, namely relating to tumours and inflammation. For instance large unilamellar liposomes LUV may be used.

Many targeting entities (called biovectors) can be used, notably those mentioned in WO 2004/112839 notably pages 65 to 82, in particular among the followings :
1) Biovectors described in documents WO 01/97850 (targeting VEGF receptors and angiopoietin), US 6,372,194 (polymer such as polyhystidine), WO 2001/9188 (fibrin-targeting polypeptide), WO 01/77145 (integrin-targeting peptide), WO 02/26776 (alphavbeta3 integrin-targeting peptide), WO 99/40947 (peptides targeting, for example, the KDR/Flk-I receptor, including R-X-K-X-H and R-X-K-X-H, or the Tie-1 and 2 receptors), WO 02/062810 and « Müller et al, Eur.J.Org.Chem , 2002,3966-3973 (glycosides of sialyl Lewis), WO 03/011115 (peptide with chelates coupled to the N and C terminal ends), Bioorganic&medicinal Chemistry letters 13,2003,1709-1712 (polyacrylamide targeting P selectine), Bioorganic&medicinal Chemistry letters 14,2004,747-749 (4-nitroimidazoles targeting tumors, WO 02/40060 (antioxidants such as ascorbic acid), US 6,524,554 (targeting of tuftsin), WO 02/094873 (targeting of G- protein receptors GPCRs, in particular cholecystokinin), US 6,489,333 (integrin antagonist and guanidine mimetic combination), US 6,511,648 (quinolone targeting alphavbeta3 or alphavbeta5), US A 2002/0106325, WO 01/97861 (benzodiazepines and analogues targeting integrins), WO 01/98294 (imidazoles and analogues), WO 01/60416 (MMP inhibitors, in particular hydroxamates), WO 02/081497 (alphavbeta3-targeting peptides such as RGDWXE), WO 01/10450 (RGD peptides), US 6,261,535 (antibodies or antibody fragments (FGF, TGFb, GV39, GV97, ELAM, VCAM, inducible with TNF or IL)), US 5 707 605 (targeting molecule modified by interaction with its target), WO 02/28441 (amyloid-deposit targeting agents), WO 02/056670 (cathepsin-cleaved peptides), US 6,410,695 (mitoxantrone or quinone), US 6,391,280 (epithelial-cell-targeting polypeptides), US 6,491,893 (GCSF), US 2002/0128553, WO 02/054088, WO 02/32292, WO 02/38546, WO 2003/6059, US 6,534,038, WO 99/54317 (cysteine protease inhibitors), WO 0177102, EP 1 121 377, Pharmacological Reviews (52, n°2, 179; growth factors PDGF, EGF, FGF, etc.), Topics in Current Chemistry (222, W. Krause, Springer), Bioorganic & Medicinal Chemistry (11, 2003, 1319-1341; alphavbeta3-targeting tetrahydrobenzazepinon derivatives).
2) Angiogenesis inhibitors, in particular those tested in clinical trials or already commercially available, especially:
   - antiogenesis inhibitors involving FGFR or VEGFR receptors, such as SU101, SU5416, SU6668, ZD4190, PTK787, ZK225846, azacycle compounds (WO 00/244156, WO 02/059110);
   - angiogenesis inhibitors involving MMPs, such as BB25-16 (marimastat), AG3340 (prinomastat), solimastat, BAY12-9566, BMS275291, metastat, neovastat;
   - angiogenesis inhibitors involving integrins, such as SM256, SG545, EC-ECM-blocking adhesion molecules (such as EMD 121-974, or vitaxin);
   - medicinal products with a more indirect mechanism of antiangiogenesis action, such as carboxiamidotriazole, TNP470, squalamine, ZD0101;
   - the inhibitors described in document WO 99/40947, monoclonal antibodies very selective for binding to the KDR receptor, somatostatin analogues (WO 94/00489), selectin-binding peptides (WO 94/05269), growth factors (VEGF, EGF, PDGF, TNF, MCSF, interleukins); VEGF-targeting biovectors described in Nuclear Medicine Communications,1999, 20;
   - the inhibitory peptides of document WO 02/066512.
3) Biovectors capable of targeting receptors: CD36, EPAS-1, ARNT, NHE3, Tie-1, 1/KDR, Flt-1, Tek, neuropilin-1, endoglin, pleiotrophin, endosialin, Axl., alPi, a2ssl, a4P1, a5pl, eph B4 (ephrin), laminin A receptor, neutrophilin 65 receptor, OB-RP leptin receptor, CXCR-4 chemokine receptor (and other receptors mentioned in document WO 99/40947), LHRH, bombesin/GRP, gastrin receptors, VIP, CCK, Tln4.
4) Biovectors of the tyrosine kinase inhibitor type.
5) Known inhibitors of the GPIIb/IIIa inhibitor selected from: (1) the fab fragment of a monoclonal antibody for the GPIIb/IIIa receptor, Abciximab (ReoPro™), (2) small peptide and peptidomimetic molecules injected intravenously, such as eptifibatide (Integrilin™) and tirofiban (Aggrastat™).
6) Peptides which are fibrinogen receptor antagonists (EP 425 212), peptides which are IIb/IIIa receptor ligands, fibrinogen ligands, thrombin ligands, peptides capable of targeting atheroma plaque, platelets, fibrin, hirudin-based peptides, guanine-based derivatives which target the IIb/IIIa receptor.
7) Other biovectors or biologically active fragments of biovectors known to the person skilled in the art as medicinal products, with antithrombotic action, antiplatelet aggregation action, action against atherosclerosis, action against restenosis, and/or anticoagulant action.
9) Some biovectors which target specific types of cancer, for example peptides which target the ST receptor associated with colorectal cancer, or the tachykinin receptor.
10) Biovectors which use phosphine-type compounds.
11) Biovectors for targeting P-selectin, E-selectin (for example the 8-amino acid peptide described by Morikawa et al, 1996, 951).
12) Annexin V and any derivatives thereof
13) Any peptide obtained by targeting technologies such as phage display, optionally modified with unnatural amino acids (http//chemlibrary.bri.nrc.ca), for example peptides derived from phage display libraries: RGD, NGR, CRRETAWAC, KGD, RGD-4C, XXXY*XXX, RPLPP, APPLPPR.
14) Other known peptide biovectors for targeting atheroma plaques, mentioned in particular in document WO 2003/014145.
15) Vitamins.
16) Ligands for hormone receptors, including hormones and steroids.
17) Opioid receptor-targeting biovectors.
18) TKI receptor-targeting biovectors.
19) LB4 and VnR antagonists.
20) Nitriimidazole and benzylguanidine compounds.
21) Biovectors recalled in Topics in Current Chemistry, vol.222, 260-274, Fundamentals of Receptor-based Diagnostic Metallopharmaceuticals, in particular:
22)biovectors for targeting peptide receptors overexpressed in tumours (LHRH receptors, bombesin/GRP, VIP receptors, CCK receptors, tachykinin receptors, for example), in particular somatostatin analogues or bombesin analogues, optionally glycosylated octreotide-derived peptides, VIP peptides, alpha-MSHs, CCK-B peptides;
   peptides selected from: cyclic RGD peptides, fibrin-alpha chain, CSVTCR, tuftsin, fMLF, YIGSR (receptor: laminin).
23) Biovectors used for products of the smart type, for instance biovectors clivable in case of biochemical local reaction namely enzymatic.
24) Markers of myocardial viability (tetrofosmin and hexakis-2-methoxy-2-methylpropyl isonitrile).
25) Ligands for neurotransmitter receptors (D, 5HT, Ach, GABA, NA receptors).
26) tyrosine kinase inhibitors, for instance Gefitinib, Erlotinib, Imatinib Oligonucleotides.
27) antibodies known for their tumoral targeting.
Other biovectors such as those described in WO2005/049005, WO2005/049095 WO2005/042033, moiety targeting amyloid beta plaques (alzheimer) such as derivative of thioflavine or chrysamine G ou red congo

The specific targeting CEST agents can also include at least two different encapsulating systems having different targeting moities and metals (cocktail of encapsulating systems containing different targeting biovectors and cest agents). For instance a diagnostic agent can comprise a mixture of i) ES/liposomes incorporating Tm3+ and a biovector specific for metalloprotease MMP1 and ii) liposomes with Dy3+ and a biovector for metalloprotease MMP9. The targeting moieties may target ligands associated to different mechanisms, for instance associated to angiogenesis and ligands associated to targets. CEST systems of the invention can be also used for stem cells taging.

To produce the contrast media compositions of the invention, the lipasomes are formulated in pharmaceutically physiologically tolerable liquid carrier medium, eg. an aqueous solution which may include one or more additives, such as pH modifying agents, chelating agents, antioxidants, tonicity modifying agents, cryoprotectants, further contrast agents, etc.

Examples of suitable ingredients to adjust the pH, include physiologically tolerable acids, bases and buffers, such as acetic acid, citric acid, fumaric acid, hydrochloric acid, malic acid, phosphoric acid, sulfuric acid, or tartaric acid, ammonia, ammonium carbonate, diethanolamine, diisopropanolamine, potassium hydroxide, sodium bicarbonate, sodium borate, sodium carbonate, sodium hydroxide, trolamine, ammonium phosphate, boric acid, citric acid, lactic acid, potassium metaphosphate, potassium phosphate monobasic, sodium acetate, sodium biphosphate, sodium citrate, sodium lactate, sodium phosphate, Tris, and N-methyl glucamine.

Examples of suitable anti-oxidants include ascorbic acid, ascorbyl palmitate, cysteine, monothioglycerol, butylated hydroxyanisole, butylated hydroxytoluene, hypophosphoric acid, propyl gallate, sodium bisulfate, sodium formaldehyde sulfoxylate, sodium metabisulfate, sodium thiosulfate, sulfur dioxide, or tocopherol. Examples of suitable tonicity agents, include sodium chloride, glucose, sucrose, mannitol, sorbitol and dextrose. These agents preferably are used to make the formulation isotonic or near isotonic with blood.

Examples of suitable anti-microbial agents include, benzalkonium chloride, benzyl alcohol, chlorobutanol, metacresol, methyl p-hydroxybenzoate, propyl p-hydroxybenzoate, and timerosal.

The responsive paramagnetic agent for use in the encapsulating systems of the invention includes at least one chelated complex of a paramagnetic metal ion adapted for CEST imaging. The paramagnetic metal ion is a lanthanide (III) metal ion which has an electronic relaxation time suitably short to significantly affect the chemical shift value of the mobile protons to be irradiated for CEST. Preferred are paramagnetic metal ions selected in the group consisting of cerium (III), praseodymium(III), neodymium (III), gadolinium (III), dysprosium (III), erbium (III), terbium (III), holmium (III), thulium (III), ytterbium (III), and europium (III). Particularly preferred are Dy3+, Tb3+, Tm3+,Yb3+, Eu3+.

Examples of suitable chelating agents include DOTA, DTPA, DTPA-BMA, BOPTA, DO3A, PCTA, derivatives thereof (described for instance in Mini Reviews in Medicinal Chemistry, 2003, vol 3, n°8), and salts and complexes thereof especially calcium, sodium or meglumine salts, eg. edetate disodium, edetic acid, calcium EDTA.

Preferred paramagnetic complexes for use in the method of the invention are the chelates of the macrocyclic of the 1,4,7,10-tetraazacyclododecane-1,4,7,10-tetraacetic acid (DOTA) and their tetra amide derivatives as for example described in Accounts of Chemical Research, 2003, 36, 10, 783-790. or of 1,4,7,10-tetraazacyclododecane-1,4,7,10-tetraphosphonic acid (DOTP) or of the 1,4,7,10-tetraazacyclododecane-1,4,7-triacetic acid (DO3A) or HPDO3A or DO3AB and their tri amide derivatives or of PCTA and their tri amide derivatives or of the HOPO and their derivatives with the preferred metal ions indicated above. Preffered lanhtanides are Yb(III), Tm(III), Er(III), Ho(III), Dy(III) and Eu(III). Chelate(s) may be also chosen among Yb(III) DOTAM-Gly an Eu(III) DOTAM-Gly or Tm-DOTAM-Gly and Eu-DOTAM-Gly described in WO9625955, DOTMA-Tm or DOTMA-Dy or DOTA-Dy or DOTA Tm or HPDO3A-Dy or HPDO3A-Tm.

The invention also describes a screening method, and the compounds obtained thereof, for the identification of efficient compounds comprising a chelate shift agent and a metal, said method comprising coupling the chelate metal complex in a ES, carrying CEST imaging, and selecting the compounds.

A composition useful in the method of the invention may comprise ES/liposomes comprising a first chelate of a metal paramagnetic ion and liposomes comprising another chelate of a metal paramagnetic ion.

Parameters of interest include non exclusively temperature, pH, metabolite concentration, 02 or CO2 partial pressure, enzymatic activity, in a human or animal body organ or tissue.

The pharmaceutical preparations according to the invention can be suitably injected intravasally (for instance intravenously, intraarterially, intraventricularly, and so on) or used by way of intrathecal, intraperitoneal, intralymphatic, intracavital, oral or enteral administration.

The injectable pharmaceutical formulations are typically prepared by dissolving the active ingredient(s) and the pharmaceutically acceptable excipients in water of suitable purity from the pharmacological point of view. The resulting formulation is suitably sterilised and can be use as such or it can alternatively be lyophilised and reconstructed before the use.

These formulations can be administered in concentrations depending on the diagnostic requirements, at a dose ranging from 0.01 to 0.5 mmol/kg body weight.

Imaging parameters can be as follows:
- high resolution work carried out on a Bruker Avance 300 spectrometer operating at 7.05 T
- saturation transfer experiments carried out at 312 DEG K by irradiating the sample with a continuous wave presaturation square pulse (power of 1050 Hz) or by using a proper train of e-burp1 selective pulses.
- NMR imaging performed using a 7.05 T Bruker PharmaScan having actively shielded gradients 300 mT/m) and running ParaVison 2.1.1 software.
- Standard PDW (proton density weighted images) obtained using a SE (spin-echo) imaging sequence (using Hermite shaped 90 DEG and 180 DEG pulses).
- NMR image adopted parameters (TR/TE/NE= 3.0s/18.3ms/1); FOV (Field Of View) 30x30 mm<2>; slice thickness 2mm and image matrix 256x256 points. A 2.25 Watt square shaped saturation pulse applied for 4 s in the pre-detay of the spin-echo sequence. Two images acquired, one with saturation of the amide protons at - 4794 Hz from bulk water protons and the other with the rf irradiation offset at 4794 Hz.

Other fields may be used such as 1, 1.5 T to 3 Tesla.

### Example 1

A chelate DOTA or DOTAMGly or DOTAM as shift agent is entrapped within a liposome encapsulating system obtained from a mixture of saturated and unsaturated phospholipids and cholesterol sodium salts. For instance 50:10:40 or 55:15:30 w/w/w of dipalmitoylphosphatidyl glycerol DPPG/ oleoyl palmitoyl phosphatidylcholine / cholesterol.

Liposome prepared using solutions of DOTA of 0.1 to 1 M, allow to obtain a concentration of DOTA inside the liposomes of 0.05 to 0.5 M. The liposome prepared have a concentration of shift agent in the range 0.5 to 5 mM, the concentration of liposomes being for instance in the range 1 to 5 nM, preferably 2-4 nM. The mean diameter of liposomes is around 0.2 to 0.3 µm, notably depending of the cholesterol ratio. Imaging parameters: Bruker 7 T, 312 DEG K, 3s irradiation time. The field required to obtain good results is much lower than for liposomes containing the shift agent than for a shift agent not entrapped in liposomes. Contrast CEST images can be obtained even at a concentration of liposome in the order of 0.05 nM or even less according to the concentration of shift agent used in the liposomes.

**Example 2:** DOTA or DTPA lipophilic derivatives are prepared according to prior art : US 6 045 821 (macrocyclic chelates notably DO3A-succinyl-PE, DO3A-glutaryl-PE, DO3A-DOBA, DO3A-DOmBA, DO3A-DOoBA DO3A-DOIA, DO3A-HOBA, DO3A-OOBA and AE-DO3A-dodecenyl-PE ; see examples 3 to 24), US 5 154 914 (DTPA carrying lipophilic chains C1-C30), US 5 312 617 (DTPA with to lipophilic groups CH₂CONR).

The chelate shift agent is coupled to the phospholipidic membrane. The chelate is located either at the external face of the liposome membrane ("external chelate") or at the internal face ("internal chelate").

Liposomes were prepared by the thin film method as follows: DSPC, the lipophile Tm complexe, FA-PEG-DSPE and cholesterol were co-dissolved in chloroform/methanol mixture 1:1 (v/v) and were evaporated to dryness under reduced pressure. The total amount of lipids was typically 120µmol composed of:
- 10 to 30% of lipophile Tm complexes
- 30 to 50% of DSPC
- 20 to 40% of cholesterol
- 5 to 15% of FA-PEG-DSPE

The lipid film was then rehydrated with 3ml of buffer at pH 6.5 constituted by 20 mM HEPES and 135 mM NaCl. The resulting liposomes were gently shaken above the transition temperature (Tc = 58°C) during 2h. Next, the lipid dispersion wad extruded sequentially ten times through 400 then 100 nm polycarbonate membrane filters.

The phospholipids concentration was determined by phosphorus analysis according Rouser (1970). The efficiency of Tm chelate incorporation was analysed by inductively coupled plasma atomic emission spectroscopy (ICP-AES)

The mean diameter of the liposomes was measured at 90° angle (25°C) by photon correlation spectroscopy (PCS) with a Malvern 4700 system : around 120nm.

In order to increase the rate of "internal chelate", the preparation method may include a step of transmetallation of the "external chelate". In this method, a transmetallation solution is added to the composition comprising the liposomes displaying both "internal chelate" and "external chelate". The transmetallation solution comprises La 3+ and allows to complex the metal of the "external chelate". Thus the remaining liposomes functionally speaking contain only or substantially only "internal chelate" carrying the metal shift agent. The "external chelate" is in terms of CEST shift effect desactivated.

### Example 3: use of PCTA derivated lipophilic compound

Dysprosium and thullium complex of the ester of acid octadecanoic3-({2-[4-(3,9-bis-carboxymethyl-3,6,9,15-tetraaza-bicyclo[9.3.1]pentadeca-1(14),11(15), 12-trien-6-yl)-4-carboxy-butyrylamino]-ethoxy}-hydroxy-phosphoryloxy)- 2-octad6canoyloxy-propylic 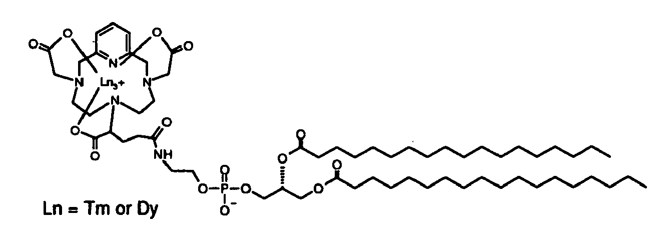 200 mg of compound 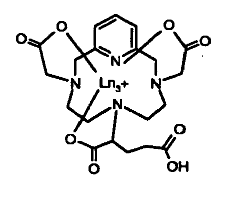 Ln = Tm or Dy
are dissolved in 10 ml of Dimethylformamide. To this solution are added 204 mg of N,N'-dicyclohexylcarbodiimide and 40mg of N-hydroxysuccunimide. The mixture is steered 1 h at room temperature and a solution of 250 mg of 1,2-Distearoyl-sn-Glycero-3-Phosphoethanolamine (DSPE, AVANTI^{®} Polar Lipids, Inc.) in 5 ml of pyridine is added. The reactive medium is steered 20h at room temperature and precipitated in 50 ml ethanol The product is then purified on silica gel. m= 190mg.

### Example 4: liposome preparation of DTPA derivatives, with PEG inserted furtive agents

### The chelate complexes are encapsulated within the liposomes, in the aquous internal phase :

Liposomes were prepared by the thin film method as follows: phospholipids (DPPC and DPPG), FA-PEG-DSPE and cholesterol were co-dissolved in chloroform/methanol mixture 5:1 (v/v) and were evaporated to dryness under reduced pressure. The total amount of lipids was typically composed of:
- 40 to 70% of DPPC
- 5 to 20% of DPPG
- 20 to 40% of cholesterol
- 5 to 15% of FA-PEG-DSPE

The lipid film was then rehydrated with an aqueous solution of 0,25M Dy-DTPA-BMA. The multilamellar vesicles were treated by freeze-thaw five times. The resulting large oligolamellar vesicles liposomes were extruded under pressure through double polycarbonate membrane filters (100 and 50 nm pore diameter). Unentrapped chelate of Dy was removed by dialysis with Spectra/Por membrane (molecular weight cut off: 10000 daltons) against buffer pH 7,4 constituted by 20 mM HEPES and 135 mM NaCl.

The phospholipids concentration was determined by phosphorus analysis according Rouser (1970). The efficiency of Dychelate incorporation was analysed by inductively coupled plasma atomic emission spectroscopy (ICP-AES)
The mean diameter of the liposomes was measured at 90° angle (25°C) by photon correlation spectroscopy (PCS) with a Malvern 4700 system : around 110nm.

### Example 5: lipophilic non peptidic (folic acid derivatives) biovector coupled to encapsulating systems

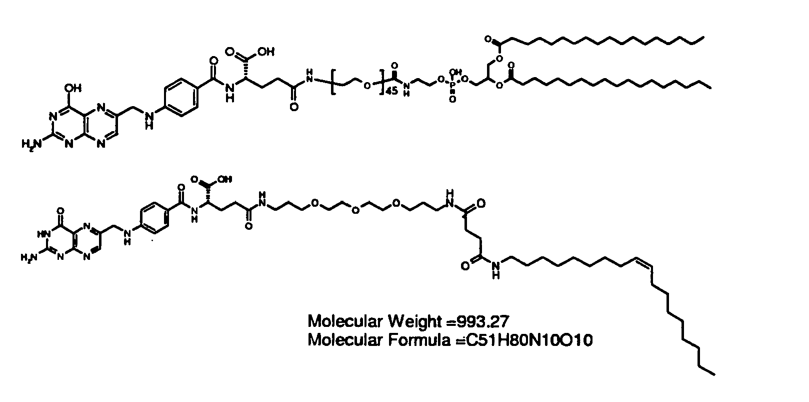

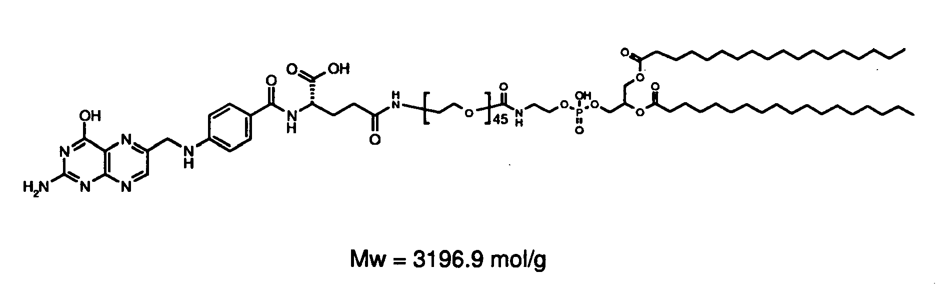

Folic acid (188mg) was dissolved in 8ml of DMSO. 700 mg of amino-PEG2000-DSPE and 3.8ml of pyridine were added followed by 233mg of dicyclohexylcarbodiimide. The reaction was continued at room temperature overnight. Pyridine was removed by rotary evaporation then 530ml of water was added. The solution was centrifuged (4000 tr/min) to remove trace insolubles. The supernatant was concentrated and ultrafiltrated on 1Kd membrane against saline solution 0.1 N first then with water. The filtrate was lyophilized and the residue dried in vacuo. The yield was 600mg (75%).Analysis: SM (electrospray positif mode): m/z with z =2 is centered on 1599, with z = 3 is centered on 1066 and with z = 4 centered on 800

### Example 6: lipophilic peptidic biovector

### step 1 :

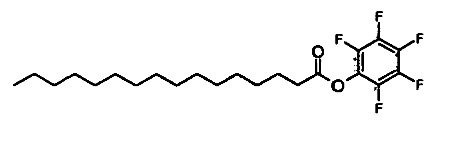

To a solution consisting of 3.85g of acid hexadecanoic and 2.76g of pentafluorophenol in 20ml of dioxan and 8ml of DMF, are added at 0°c, 3.1g of dicyclohexylcarbodiimide (DCC) in 20ml of dioxan. The mixture is steered one night at room temperature and the reactive medium is filtered. The solution obtained is evaporated at low pressure. The oil obtained is put into cyclohexan in order to obtain a white solid (5g).
Fusion temperature: 42-44°c

### Step 2 : Acylation of peptides

Peptides are prepared on solide phase by using 2.5 equivalents of each aminoacid protected Fmoc at each coupling cycle. The activation of the carboxylic acids is made with HATU, N-methylmorpholine in DMF. The Fmoc groups are coupled by a piperidine treatment (20% in DMF). After introduction of the last aminoacid of the peptidic sequence and clivage of the protecting group Fmoc, the N-terminal amine of the peptide is acylated by the compound prepared at step 1 (2.5 équivalents) dissolved in CH₂Cl₂ with presence of HoBt and N-methylmorpholine. The peptide is afterwards liberated from the resin and the protecting groups of the lateral functions are cut by action of a mixture of acid trifluoroacetique/thioanisole (95/5) during 30 minutes art 0°c and followed by 2h at room temperature. The resin is eliminated and the solvant is evaporated at low pressure. The lipopeptide is precipitated in ether ethylic. The products are purified by HPLC preparative on column Vydac ODS® by eluting with a mixture water/acetonitrile/TFA.

| **Structure** | **Moclecular weight** | **ES+ m/z** |
|---|---|---|
| 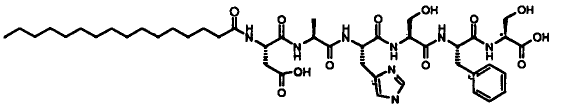 | 901.08 | 902.5 |
| 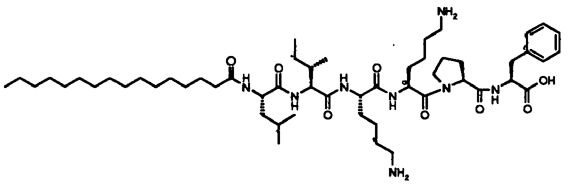 | 983.40 | 986 |
| 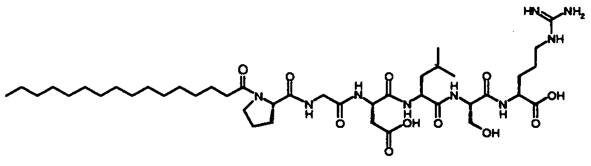 | 882.12 | 884 |

### Example 7 : furtive agent phospholipide-PEG

Such lipophilic PEG derivatives are well known, commercialy available for instance from AVANTI®polar lipids on Avantilipids.com, notably PEG 350, 750, 2000, 3000, 5000, in particular:
- 1,2-Distearoyl-sn-Glycero-3-Phosphoethanolamine-N-[Methoxy(Polyethylene glycol)-350]
- 1,2-Distearoyl-sn-Glycero-3-Phosphoethanolamine-N-[Methoxy(Polyethylene glycol)-550],
- 1,2-Distearoyl-sn-Glycero-3-Phosphoethanolamine-N-[Methoxy(Polyethylene glycol)-750]
1,2-Distearoyl-sn-Glycero-3-Phosphoethanolamine-N-[Methoxy(Polyethylene glycol)-3000].
Preferably the purcentage of PEGylated phospholipid is in the order of 10.

The invention is not limited to the encapsulating systems described in detail. In particular the invention includes any CEST ES agent comprising a lipidic composition mentioned of the ES and a chelate shift agent which displays effective good results analogous to those described in detail, by appropriate CEST protocol.

### Example 8: preparation of multilamellar liposomes

Multilamelar liposomes (or MLV, multilamelar vesicule) were prepared by the thin film method as follows: phospholipids (DPPC and DPPG), FA-PEG-DSPE and cholesterol were co-dissolved in chloroform/methanol mixture 5:1 (v/v) and were evaporated to dryness under reduced pressure. The total amount of lipids was typically composed of:
- 40 to 70% of DPPC
- 5 to 20% of DPPG
- 20 to 40% of cholesterol
- 5 to 15% of FA-PEG-DSPE

The lipid film was then rehydrated with an aqueous solution of 0.25M Dy-DOTA or Tm-DOTA. Unentrapped chelat of Dy or Tm was removed by dialysis with Spectra/Por membrane (molecular weight cut off : 10000 daltons) against buffer pH 7.4 constituted by 20 mM HEPES and 135 mM NaCl.

The phospholipids concentration was determined by phosphorus analysis according Rouser (1970). The efficiency of Dy ot Tm chelate incorporation was analysed by inductively coupled plasma atomic emission spectroscopy (ICP-AES) The mean diameter of the liposomes was measured at 90° angle (25°C) by photon correlation spectroscopy (PCS) with a Malvern 4700 system: around 600 nm.

### Comparative Example 9: preparation of double emulsion

The W/O/W double microemulsion was prepared in two steps. A warm W/O microemulsion was first prepared by adding an aqueous solution (3 to 10%) at 70°C containing the Dy-DOTA (0.05-0.2 M) to a mixture of:
- Stearic acid (35 to 80 %)
- AOT (10 to 20%)
- Butanol (20 to 30%)

The W/O/W double emulsion was obtained by adding to the warm W/O microemulsion (10 to 15%) a mixture warmed at 70°C of:
- Water (50 to 70%)
- Lecithin (3 to 10%)
- Butanol (5 to 10%)
- taurodeoxycholate sodium salt (TDC) (5 to 10%)

Both steps resulted in the formation of optically transparent systems.
The W/O/W microemulsion was purified three times by ultradiafiltration, using Diaflo membranes. The efficiency of Dy ot Tm chelate incorporation was analysed by inductively coupled plasma atomic emission spectroscopy (ICP-AES)

## Claims

1. Use of a CEST contrast agent in a method of CEST imaging, wherein the contrast agent comprises a proton pool encapsulating system, which is a liposome, that contains 1) a pool of water mobile protons to be shifted and 2) a paramagnetic complex of a lanthanide, with the exception of [(SR) (SH)] adduct compartmentalised in a liposome in which SR is Ln [DOTP]⁴⁻ wherein Ln is a lanthanide selected from Cerium (III), Praseodymium (III), Neodymium (III), Dysprosium (III), Erbium (III), Terbium (III), Holmium (III), Thulium (III), Ytterbium (III) and Europium (III) and SH is a diamagnetic substrate selected from linear and cyclic polyamines, polyaminoacids, proteins, polysaccharides, polyamidoamines, peramidated polyaminoacids, dendrimers containing amide groups, polycyclodextrins, polysaccharides and alginates .

2. Use of a CEST contrast agent in a method of CEST imaging, wherein the contrast agent comprises a proton pool encapsulating system which is a liposome, that contains 1) a pool water mobile protons to be shifted and 2) a paramagnetic complex of a lanthanide, the encapsulating system structure being adapted for active targeting to specific organs or tissues by incorporation of lipids with attached thereto biovectors chosen between monoclonal antibodies or antibody fragments that are specific for tumour associated antigens, lectins and peptides.

3. The use of claim 1 or 2 wherein the chelating group of the paramagnetic complex is chosen among DOTA, DTPA, DTPA-BMA, BOPTA, DO3A, PCTA, derivatives of DOTA, DTPA, DTPA-BMA, BOPTA, PCTA and DO3A, DOTAMgly, the tris amide derivatives of DO3A and PCTA and the tetra amide derivatives of DOTA.

4. The use of claim 1-3 wherein said lanthanide is chosen among Erbium (II), Europium (III), Dysprosium (III), Gadolinium (III), Praseodymium (III), Neodymium (III), Terbium (III), Holmium (III), Thulium (III), Ytterbium (III).

5. The use of claims 4 wherein said lanthanide is chosen among Europium (III), Dysprosium (III) and Ytterbium (III).

6. The use of claim 4 wherein said paramagnetic complex is a chelate chosen among: Yb(III) DOTAM-Gly, Tm(III)-DOTAM-Gly and Eu-DOTAM-Gly.

7. The use of claims 1-6 wherein the encapsulating sytem forming lipids comprise phospholipids or hydrogenated phospholipids or derivatives thereof among phosphatidylcholines, phosphatidylethanolamines, lysolecithins, lysophosphatidylethanolamines, phosphatidylserines, phosphatidylglycerols, phosphatidylinositol, sphingomyelins, cardiolipin, phosphatidic acids, fatty acids, gangliosides, glucolipids, glycolipids, mono-, di or triglycerides, ceramides or cerebrosides.

8. The use of claims 1-7 wherein steroids, notably cholesterol, cholesterol derivatives, cholestane, cholic acid and bile acids, are incorporated into the liposomal membrane.

9. The use of claims 1-7 wherein the liposome include 1 to 10 % relative to the weight of liposome membrane forming material of negatively charged phospholipids.

10. The use of claims 1-10 wherein the liposome comprises 1 -10% of PEG-PE derivatives relative to the weight of the liposome membrane forming material.

11. The use of claims 1-10 wherein the liposome is of diameter of range 20-5000 nm.

12. The use of claims 1 to 8 wherein the chelate is tethered to the liposomal membrane.

13. The use of claim 12 wherein the chelate is coupled to anchor molecules in the liposomes.

14. The use of claims 1 to 13 wherein the contrast agent comprises liposomes comprising a first chelate of a metal paramagnetic ion and liposomes comprising another chelate of a metal paramagnetic ion.

15. The use of claims 1 to 13 wherein the encapsulating system structure is adapted for blood pool imaging.

16. The use of claims 1 to 15 wherein the liposome is an envirosensitive liposome.

17. A method for the determination by MRI of a chemico-physical parameter in a human or animal body organ, fluid or tissue, wherein a CEST agent as defined in claims 1 to 16 is employed whose saturation transfer capability is correlated to the chemico- physical parameter of interest and a CEST MRI image for this chemico- physical parameter is registered.

18. A method for the determination by MRI of a chemico-physical parameter comprising the administration in appropriate quantity of at least one contrast agent as defined in claims 1 to 16, said agent comprising a pool of mobile protons in chemical exchange with the water medium protons and able, when a proper radiofrequency rf irradiating field is applied at the resonance frequency of the said exchangeable protons pool, to generate a saturation transfer effect between at least a part of said mobile pool of protons and the water protons and wherein said saturation transfer relates to the chemico-physical parameter of interest.

## Patentansprüche

1. Verwendung eines CEST-Kontrastmittels bei einem Verfahren der CEST-Bildgebung, wobei das Kontrastmittel ein Protonenpool-verkapselndes System umfasst, welches ein Liposom ist, das 1) einen Pool von zu verschiebenden, mobilen Protonen vom Wasser, und 2) einen paramagnetischen Komplex eines Lanthanids, mit Ausnahme von [(SR)(SH)]-Addukt, kompartimentalisiert in einem Liposom, wobei SR Ln[DOTP]⁴⁻ ist, wobei Ln ein Lanthanid ist, ausgewählt aus Cer(III), Praseodym(III), Neodym(III), Dysprosium(III), Erbium(III), Terbium(III), Holmium(III), Thulium(III), Ytterbium(III) und Europium(III), und SH ein diamagnetisches Substrat ist, ausgewählt aus linearen und cyclischen Polyaminen, Polyaminosäuren, Proteinen, Polysacchariden, Polyamidoaminen, peramidierten Polyaminosäuren, Dendrimeren, die Amidgruppen enthalten, Polycyclodextrinen, Polysacchariden und Alginaten, enthält.

2. Verwendung eines CEST-Kontrastmittels bei einem Verfahren der CEST-Bildgebung, wobei das Kontrastmittel ein Protonenpool-verkapselndes System umfasst, welches ein Liposom ist, das 1) einen Pool von zu verschiebenden, mobilen Protonen vom Wasser und 2) einen paramagnetischen Komplex eines Lanthanids enthält, wobei die Struktur des verkapselnden Systems für das aktive Anzielen von spezifischen Organen oder Geweben durch Einverleiben von Lipiden mit daran befestigten Biovektoren, ausgewählt aus monoklonalen Antikörpern und Antikörperfragmenten, die für tumorverbundene Antigene spezifisch sind, Lectinen und Peptiden, ausgelegt ist.

3. Verwendung gemäß Anspruch 1 oder 2, wobei die chelatbildende Gruppe des paramagnetischen Komplexes ausgewählt ist aus DOTA, DTPA, DTPA-BMA, BOPTA, D03A, PCTA, Derivaten von DOTA, DTPA, DTPA-BMA, BOPTA, PCTA und D03A, DOTAMgly, den Trisamidderivaten von D03A und PCTA sowie den Tetraamidderivaten von DOTA.

4. Verwendung gemäß Ansprüchen 1-3, wobei das Lanthanid ausgewählt ist aus Erbium(II), Europium(III), Dysprosium(III), Gadolinium(III), Praseodym(III), Neodym(III), Terbium(III), Holmium(III), Thulium(III), Ytterbium(III).

5. Verwendung gemäß Anspruch 4, wobei das Lanthanid ausgewählt ist aus Europium(III), Dysprosium(III) und Ytterbium(III).

6. Verwendung gemäß Anspruch 4, wobei der paramagnetische Komplex ein Chelat ist, ausgewählt aus Yb(III)-DOTAM-Gly, Tm(III)-DOTAM-Gly und Eu-DOTAM-Gly.

7. Verwendung gemäß Ansprüchen 1-6, wobei die Lipide, die das verkapselnde System bilden, Phospholipide oder hydrierte Phospholipide oder Derivate davon aus Phosphatidylcholinen, Phosphatidylethanolaminen, Lysolecithinen, Lysophosphatidylethanolaminen, Phosphatidylserinen, Phosphatidylglycerolen, Phosphatidylinositol, Sphingomyelinen, Cardiolipin, Phosphatidinsäuren, Fettsäuren, Gangliosiden, Glucolipiden, Glycolipiden, Mono-, Di- oder Triglyceriden, Ceramiden oder Cerebrosiden umfassen.

8. Verwendung gemäß Ansprüchen 1-7, wobei Steroide, insbesondere Cholesterol, Cholesterolderivate, Cholestan, Cholsäure und Gallensäuren, in die liposomale Membran einverleibt sind.

9. Verwendung gemäß Ansprüchen 1-7, wobei das Liposom 1 bis 10 Gew.-%, bezogen auf das Liposomenmembran-bildende Material, an negativ geladenen Phospholipiden umfasst.

10. Verwendung gemäß Ansprüchen 1-10, wobei das Liposom 1-10 Gew.-% PEG-PE-Derivate, bezogen auf das Liposomenmembran-bildende Material, umfasst.

11. Verwendung gemäß Ansprüchen 1-10, wobei das Liposom einen Durchmesser im Bereich von 20-5000 nm aufweist.

12. Verwendung gemäß Ansprüchen 1 bis 8, wobei das Chelat an die liposomale Membran angebunden ist.

13. Verwendung gemäß Anspruch 12, wobei das Chelat an Ankermoleküle in den Liposomen gekoppelt ist.

14. Verwendung gemäß Ansprüchen 1 bis 13, wobei das Kontrastmittel Liposomen, die ein erstes Chelat eines paramagnetischen Metallions umfassen, und Liposomen, die anderes Chelat eines paramagnetischen Metallions umfassen, umfasst.

15. Verwendung gemäß Ansprüchen 1 bis 13, wobei die Struktur des verkapselnden Systems für die Blutpool-Bildgebung ausgelegt ist.

16. Verwendung gemäß Ansprüchen 1 bis 15, wobei das Liposom ein umgebungsempfindliches Liposom ist.

17. Verfahren zum Bestimmen eines chemisch-physikalischen Parameters in einem Organ, Fluid oder Gewebe des menschlichen oder tierischen Körpers durch MRI, wobei ein CEST-Mittel gemäß Ansprüchen 1 bis 16 eingesetzt wird, dessen Sättigungstransfervermögen mit dem betrachteten chemisch-physikalischen Parameter korreliert wird und ein CEST-MRI-Bild für diesen chemisch-physikalischen Parameter aufgezeichnet wird.

18. Verfahren zum Bestimmen eines chemisch-physikalischen Parameters durch MRI, umfassend das Verabreichen von wenigstens einem Kontrastmittel gemäß Ansprüchen 1 bis 16 in ausreichender Menge, wobei das Mittel einen Pool von mobilen Protonen in chemischem Austausch mit den Protonen des Wassermediums umfasst und fähig ist, bei Anwendung eines geeigneten, mit Radiofrequenz rf strahlenden Feldes bei der Resonanzfrequenz des Pools von austauschbaren Protonen einen Sättigungstransfereffekt zwischen wenigstens einem Teil des mobilen Protonenpools und den Wasserprotonen zu erzeugen, und wobei der Sättigunstransfer mit dem betrachteten chemisch-physikalischen Parameter in Beziehung steht.

## Revendications

1. Utilisation d'un produit de contraste CEST dans un procédé d'imagerie CEST, pour laquelle le produit de contraste comprend un système d'encapsulation d'un pool de protons, lequel est un liposome, qui contient 1) un pool de protons mobiles de l'eau à déplacer et 2) un complexe paramagnétique d'un lanthanide, à l'exception d'un adduit [(SR)(SH)] compartimentalisé dans un liposome dans lequel SR est Ln[DOTP]⁴⁻ où Ln est un lanthanide choisi parmi le cérium(III), le praséodyme(III), le néodyme(III), le dysprosium(III), l'erbium(III), le terbium(III), le holmium(III), le thulium(III), l'ytterbium(III) et l'europium(III), et SH est un substrat diamagnétique choisi parmi les polyamines linéaires et cycliques, les poly(acides aminés), les protéines, les polysaccharides, les polyamidoamines, les poly(acides aminés) peramidés, les dendrimères contenant des groupes amide, les polycyclodextrines, les polysaccharides et les alginates.

2. Utilisation d'un produit de contraste CEST dans un procédé d'imagerie CEST, pour laquelle le produit de contraste comprend un système d'encapsulation d'un pool de protons, lequel est un liposome, qui contient 1) un pool de protons mobiles de l'eau à déplacer, et 2) un complexe paramagnétique d'un lanthanide, la structure du système d'encapsulation étant apte à cibler d'une manière active des organes ou des tissus spécifiques par incorporation de lipides, auxquels sont fixés des biovecteurs choisis parmi les anticorps monoclonaux ou les fragments d'anticorps qui sont spécifiques d'antigènes associés à des tumeurs, des lectines et des peptides.

3. Utilisation de la revendication 1 ou 2, pour laquelle le groupe chélatant du complexe paramagnétique est choisi parmi DOTA, DTPA, DTPA-BMA, BOPTA, D03A, PCTA, les dérivés du DOTA, du DTPA, du DTPA-BMA, du BOPTA, du PCTA et du D03A, DOTAMgly, les dérivés trisamidés du D03A et du PCTA, et les dérivés tétraamidés du DOTA.

4. Utilisation des revendications 1 à 3, pour laquelle ledit lanthanide est choisi par l'erbium(II), l'europium(III), le dysprosium(III) le gadolinium(III), le praséodyme(III), le néodyme(III), le terbium(III), l'holmium(III), le thulium(III), l'ytterbium(III).

5. Utilisation de la revendication 4, pour laquelle ledit lanthanide est choisi parmi l'europium(III), le dysprosium(III) et l'ytterbium(III).

6. Utilisation de la revendication 4, pour laquelle ledit complexe paramagnétique est un chélate choisi parmi Yb(III)DOTAM-Gly, Tm(III)DOTAM-Gly et Eu-DOTAM-Gly.

7. Utilisation des revendications 1 à 6, pour laquelle les lipides formant le système d'encapsulation comprennent les phospholipides ou les phospholipides hydrogénés ou les dérivés de ceux-ci parmi les phosphatidylcholines, les phosphatidyléthanolamines, les lysolécithines, les lysophosphatidyléthanolamines, les phosphatidylsérines, les phosphatidylglycérols, le phosphatidylinositol, les sphingomyélines, la cardiolipine, les acides phosphatidiques, les acides gras, les gangliosides, les glucolipides, les glycolipides, les mono-, di- ou triglycérides, les céramides ou les cérébrosides.

8. Utilisation des revendications 1 à 7, pour laquelle des stéroïdes, notamment le cholestérol, les dérivés du cholestérol, le cholestane, l'acide cholique et les acides biliaires, sont incorporés dans la membrane liposomique.

9. Utilisation des revendications 1 à 7, pour laquelle le liposome comprend 1 à 10 %, par rapport au poids du matériau formant la membrane liposomique, de phospholipides négativement chargés.

10. Utilisation des revendications 1 à 10, pour laquelle le liposome comprend 1 à 10 % de dérivés de PEG-PE, par rapport au poids du matériau formant la membrane liposomique.

11. Utilisation des revendications 1 à 10, pour laquelle le liposome a un diamètre compris dans la plage de 20 à 5000 nm.

12. Utilisation des revendications 1 à 8, pour laquelle le chélate est attaché à la membrane liposomique.

13. Utilisation de la revendication 12, pour laquelle le chélate est couplé à des molécules d'ancrage dans les liposomes.

14. Utilisation des revendications 1 à 13, pour laquelle le produit de contraste comprend des liposomes comprenant un premier chélate d'un ion paramagnétique métallique et des liposomes comprenant un autre chélate d'un ion paramagnétique métallique.

15. Utilisation des revendications 1 à 13, pour laquelle la structure du système d'encapsulation est apte à réaliser une imagerie d'un pool sanguin.

16. Utilisation des revendications 1 à 15, pour laquelle le liposome est un liposome enviro-sensible.

17. Procédé de détermination par IRM d'un paramètre physico-chimique dans un organe, un fluide ou un tissu corporel humain ou animal, dans lequel un produit CEST tel que défini dans les revendications 1 à 16 est utilisé, dont le pouvoir de transfert de saturation est corrélé au paramètre physico-chimique d'intérêt, et on enregistre une image IRM du CEST pour ce paramètre physico-chimique.

18. Procédé de détermination par IRM d'un paramètre physico-chimique, comprenant l'administration, en une quantité appropriée, d'au moins un produit de contraste tel que défini dans les revendications 1 à 16, ledit produit comprenant un pool de protons mobiles en échange chimique avec les protons du milieu aqueux et apte, quand on applique un champ de rayonnement à une radiofréquence appropriée rf, à la fréquence de résonance dudit pool de protons échangeables, à réaliser un effet de transfert de saturation entre au moins une partie dudit pool mobile de protons et les protons se trouvant dans l'eau, ledit transfert de saturation étant relié au paramètre physico-chimique d'intérêt.
